# EUROPÄISCHE PATENTANMELDUNG

(11) **EP 2 926 838 A1**
(43) Veröffentlichungstag der Anmeldung: **07.10.2015**
(21) Anmeldenummer: 15154423.6
(22) Anmeldetag: 10.02.2015
(51) Int. Cl.: A61L 9/20, A61L 2/10

(54) **Vorrichtung zur Entkeimung mittels ultravioletter Strahlung**

(30) Priorität: 04.04.2014 DE 102014104851
(71) Anmelder: Heraeus Noblelight GmbH, 63450 Hanau (DE)
(72) Erfinder: Dr. Lott, Josef Zoltan, 22763 Hamburg (DE)
(74) Vertreter: Heraeus IP

(57) **Zusammenfassung**

Bei einer bekannten Vorrichtung zur Entkeimung mittels ultravioletter Strahlung ist ein UV-Strahler (4) in einem Gehäuse (2) aus Metall oder aus Kunststoff angeordnet, das ein Strahlungsaustrittsfenster (3) für die Emission von UV-Strahlung aufweist, welches von einer für ultraviolette Strahlung durchlässigen Polymerfolie (7) bedeckt ist. Um hiervon ausgehend eine zuverlässige und betriebssichere Vorrichtung zur Entkeimung zur Verfügung zu stellen, wird vorgeschlagen, dass das Gehäuse (2) einen Gaseinlass (22) für die Einleitung eines Kühlgasstromes sowie einen Gasauslass (23) für die Ableitung des Kühlgasstromes aufweist, und dass der Gasauslass (23) mit einer Messeinrichtung (17; 18, 20, 21) zur Messung von Druck, Massenstrom und/oder Volumenstrom des abgeleiteten Kühlgasstromes verbunden ist (Fig. 4).

## Beschreibung

### Technischer Hintergrund

Die vorliegende Erfindung betrifft eine Vorrichtung zur Entkeimung mittels ultravioletter Strahlung, umfassend einen UV-Strahler, der in einem Gehäuse aus Metall oder aus Kunststoff angeordnet ist, das ein Strahlungsaustrittsfenster für die Emission von UV-Strahlung aufweist, welches von einer für ultraviolette Strahlung durchlässigen Polymerfolie bedeckt ist.

### Stand der Technik

UV-Strahler werden beispielsweise in Klima- und Trinkwasseranlagen sowie in der Lebensmittelproduktion zur Entkeimung eingesetzt. Dabei werden Lebensmittel, wie Obst- und Gemüse, sowohl bestrahlt, als auch Maschinenteile, Packstoffe, Flüssigkeiten, Luft und Oberflächen, die mit dem Lebensmittel bei der Zubereitung in Kontakt kommen. Die UV-Strahler sind dabei üblicherweise von einem Quarzglas-Hüllrohr umgeben und können in unmittelbarer Nähe des zu bestrahlenden Guts oder in größerem Abstand angeordnet sein, beispielsweise als Deckenarmatur zur Luft- und Oberflächenentkeimung. Nachteilig ist, dass beim Bruch des Quarzglas-Hüllrohres Bruchstücke in oder auf das zu bestrahlende Gut gelangen können. Um dies zu verhindern, sind eine Vielzahl von Sicherheitsvorrichtungen bekannt, die, jedoch einen gewissen Mess- und Materialaufwand erfordern.

Diesen Nachteil vermeidet eine Entkeimungsvorrichtung gemäß der eingangs genannten Gattung, wie sie beispielsweise aus der DE 10 2005 026 645 A1 bekannt ist. Darin wird vorgeschlagen, den von einem Quarzglas-Hüllrohr umgebenen UV-Strahler in einem zylindrischen Metall- oder Kunststoffgehäuse aufzunehmen das mit einem Strahlungsaustrittsfenster für die Emission der UV-Strahlung ausgestattet ist. Über das Gehäuse und das Strahlungsaustrittsfenster ist eine Folie aus einem Fluorpolymer (MFA) geschrumpft. Diese weist eine hohe Durchlässigkeit für Strahlung mit Wellenlängen um 253,7 nm auf.

### Technische Aufgabe

Beim bestimmungsgemäßen Einsatz sind der UV-Strahler und das ihn umhüllende Gehäuse sowie die UV-durchlässige Kunststofffolie hohen Temperaturen, Wasser, Wasserdampf oder auch korrosiven und ätzenden Substanzen ausgesetzt und unterliegen dabei hohen thermischen, mechanischen und chemischen Belastungen.

Diese können zu Alterung und Beschädigung der Kunststofffolie führen, so dass Fremdkörper, Staub oder Flüssigkeiten in das Gehäuse eindringen und die dort vorhandenen optischen und elektrischen Bauteile und insbesondere die Leistung der UV-Lampe beeinträchtigen.

Der Erfindung liegt daher die Aufgabe zugrunde, diesen Nachteil zu vermeiden, und eine zuverlässige und betriebssichere Vorrichtung zur Entkeimung zur Verfügung zu stellen.

### Allgemeine Beschreibung der Erfindung

Diese Aufgabe wird ausgehend von einer Vorrichtung der eingangs genannten Gattung erfindungsgemäß dadurch gelöst, dass das Gehäuse einen Gaseinlass für die Einleitung eines Kühlgasstromes sowie einen Gasauslass für die Ableitung des Kühlgasstromes aufweist, und dass der Gasauslass mit einer Messeinrichtung zur Messung von Druck, Massenstrom und/oder Volumenstrom des abgeleiteten Kühlgasstromes verbunden ist.

Das den UV-Strahler vor Schmutz, Temperatur und Flüssigkeiten schützende Gehäuse ist mit einem Einlass und mit einem Auslass für das Kühlgas versehen. Mittels des Kühlgases kann der UV-Strahler auf einer vorgegebenen Temperatur gehalten werden, was auch den Einsatz von Hochleistungsstrahlern, bei denen eine Kühlung erforderlich ist, ermöglicht. Auf ein den UV-Strahler zusätzlich umgebendes Hüllrohr - wie beim oben genannten Stand der Technik - kann bei der Erfindung daher verzichtet werden.

Der Kühlgasstrom erfüllt bei der Erfindung eine weitere wesentliche Funktion. Er dient als Prüf-Gasstrom zur Feststellung einer Leckage am Gehäuse, insbesondere der Dichtheit der Polymerfolie. Zu diesem Zweck ist eine Messeinrichtung vorgesehen, die mindestens die Messung von Druck, Massenstrom und/oder Volumenstrom des ausgeleiteten Kühlgasstromes ermöglicht. Bei unerwarteten Schwankungen eines oder mehrere dieser Parameter oder bei einer Abweichung von einem vorgegebenen Sollwert kann von einer Leckage ausgegangen werden. Der Gasauslass ist mit der Messeinrichtung unmittelbar oder mittelbar - über ein oder mehrere andere Bauteile verbunden.

Das Gehäuse besteht vorzugsweise aus Metall, insbesondere aus Edelstahl. Es umgibt einen oder mehrere UV-Strahler und/oder andere Strahler. UV-Strahler in diesem Sinne sind beispielsweise Quecksilberdampf-Niederdruckstrahler, -Mittel-druckstrahler oder -Hochdruckstrahler. Vorzugsweise verlaufen die Leitungen für den elektrischen Anschluss des UV-Strahlers ebenfalls innerhalb des Gehäuses und sie werden zum Gehäuse über Zuleitungs- oder Ableitungsrohre für den Kühlgasstrom verlegt. Dadurch werden separate Gehäuse-Öffnungen für die Betriebs- und Datenleitungen vermieden, was zur Dichtigkeit beiträgt.

Es hat sich als günstig erwiesen, wenn die Messeinrichtung einen mit dem Gaseinlass verbundenen, ersten Sensor und einen mit dem Gasauslass verbundenen, zweiten Sensor umfasst.

Mittels des ersten, in Strömungsrichtung gesehenen vorderen Sensors wird der zu messende Parameter - also Volumenstrom, Massenstrom oder Gasdruck - des eingeleiteten Kühlgasstromes kontinuierlich ermittelt. Der betreffende Parameter wird auch für den abgeführten Kühlgasstrom mittels des zweiten, hinteren Sensors kontinuierlich oder von Zeit zu Zeit gemessen. Ergibt sich zwischen den beiden Messwerten eine Auffälligkeit, beispielsweise ein von einem vorgegebenen Grenzwert abweichender Druckabfall oder eine nennenswerte Differenz zwischen des Massen- oder Volumenstroms beiderseits des Gehäuses, kann von einer Leckage ausgegangen werden. Die von den Sensoren ermittelten Messwerte, insbesondere der vom vorderen Sensor ermittelte Messwert, können gleichzeitig für eine Regelung des Kühlgasstromes zwecks Temperierung des UV-Strahlers herangezogen werden.

Im einfachsten Fall ist mindestens der erste Sensor, vorzugsweise auch der zweite Sensor, als Massendurchflussregler ausgeführt.

Massendurchflussregler sind zuverlässig und genau und nicht nur zur Regelung des Gasmassendurchflusses, sondern auch zur einfachen Messung geeignet.

Dabei hat es sich als günstig erwiesen, wenn die Messeinrichtung eine Anzeigeoder Alarmeinrichtung umfasst.

Dadurch ist sichergestellt, dass eine Leckage nicht über einen längeren Zeitraum unentdeckt bleibt, sondern unmittelbar nach dem Auftreten einer Unregelmäßigkeit Gegenmaßnahmen eingeleitet werden können. Dazu kann auch ein automatisches Abschalten der Vorrichtung gehören, insbesondere wenn die Messeinrichtung eine dafür angepasste Auswerte- und Abschalteinrichtung umfasst.

Bei einer bevorzugten Ausgestaltung der erfindungsgemäßen Vorrichtung ist der UV-Strahler Teil einer innerhalb des Gehäuses angeordneten Lampeneinheit, die zwei UV-Strahler und einen IR-Strahler aufweist.

Der Infrarot-Strahler dient zur Verkürzung der Aufwärmdauer der UV-Strahler beim Start und der schnelleren Betriebsbereitschaft der Vorrichtung. Er ist vorzugsweise mittig zwischen den beiden UV-Strahlern angeordnet.

Die Lampeneinheit aus UV-Strahlern und Infrarot-Strahler bildet zusammen mit dem Kunststoff- oder Metallgehäuse ein betriebsseitig vorgefertigtes Strahlermodul. Zum Einsatz in der Entkeimungs-Vorrichtung müssen lediglich noch die Polymerfolie zur Abdeckung des Strahlungseintrittsfensters überzogen, die elektrischen Anschlüsse sowie die Gasanschlüsse für Ableitung und Zuleitung des Kühlgases hergestellt werden.

In dem Zusammenhang hat es sich als vorteilhaft erwiesen, wenn die UV-Strahler auf ihrer dem Strahlungsaustrittsfenster abgewandten Seite mit einem Reflektor versehen sind.

Bei dem Reflektor handelt es sich vorzugsweise um eine Beschichtung des Lampenkolbens der UV-Lampen mit reflektierenden Eigenschaften, beispielsweise einer Beschichtung aus opakem Quarzglas.

Das Gehäuse weist im Bereich des Strahlungsaustrittsfenster vorteilhafterweise einen länglich-ovalen oder rechteckigen Querschnitt auf.

Mindestens eine der Gehäuseseiten ist dabei weitgehend flach. Verläuft das Strahlungsaustrittsfensters vollständig im Bereich dieser Flachseite, so liegen sich die Längskanten des Strahlungsaustrittsfensters in einer gemeinsamen Ebene genau gegen. Auf diese Weise wird für die Polymerfolie eine definierte Spannebene geschaffen, die Falten und Verwerfungen der Folie entgegenwirkt.

Es hat sich bewährt, wenn die Messeinrichtung einen Temperatursensor zur Erfassung der Temperatur des UV-Strahlers umfasst.

Die Effektivität der UV-Strahlungsemission hängt maßgeblich von der Betriebstemperatur ab. Der Temperatursensor erfasst die Temperatur des UV-Strahlers fortlaufend, das heißt kontinuierlich oder von Zeit zu Zeit. Bei gleichzeitigem Einsatz mehrerer UV-Strahler kann jeder der Strahler mit einem Temperatursensor versehen sein. Alternativ kann auch nur die Temperatur an einem einzigen oder einzelnen Strahlern erfasst werden. Die Erfassung der Temperatur erfolgt vorzugsweise an der Oberfläche des Strahlerrohres. Durch die fortlaufende Erfassung der Strahler-Temperatur ist es möglich, Abweichungen der StrahlerTemperatur von einem vorgegebenen Soll-Wert schnell zu erkennen und beispielsweise durch Veränderung des Kühlgasstromes zu kompensieren.

In dem Zusammenhang ist es auch vorteilhaft, wenn der Gaseinlass mit einer Temperiervorrichtung verbunden ist, mittels welcher der Kühlgasstrom temperierbar ist.

Abweichungen der Strahlertemperatur von der nominalen Betriebstemperatur können beispielsweise auf einer hohen Umgebungstemperatur beruhen oder durch die Bauform der Bestrahlungsvorrichtung bedingt sein. Ein temperierbarer Kühlgasstrom kann auch erwärmt werden, so dass die Temperatur des UV-Strahlers schneller und genauer im gewünschten Temperaturbereich gehalten werden kann. Eine in einem Zuführungskanal für den Kühlgasstrom angeordnete Temperiervorrichtung hat den Vorteil, dass das Kühlgas in räumlicher Nähe zum UV-Strahler temperiert wird.

Die Vorrichtung kann mit einer Vielzahl von Leitungen versehen sein, beispielsweise mit Versorgungsleitungen für Medien, wie Flüssigkeiten oder Gase, mit Mess- und Datenleitungen von und zu Sensoren, Aktoren oder Rechnern oder mit Stromversorgungsleitungen.

Um Behinderungen durch diese Leitungen oder Beschädigungen der Leitungen zu verhindern, hat es sich bewährt, wenn an den Gaseinlass und/oder - vorzugsweise - an den Gasauslass ein Kühlgasführungsrohr angeschlossen ist, durch das Versorgungs- oder Datenleitungen zum hindurchgeführt sind.

### Ausführungsbeispiel

Nachfolgend wird die Erfindung anhand eines Ausführungsbeispiels und einer Zeichnung näher beschrieben. Im Einzelnen zeigt in schematischer Darstellung:
- **Figur 1**: ein Strahlermodul zum Einsatz in einer erfindungsgemäßen Entkeimungsvorrichtung mit einem Metallrohr, zwei UV-Lampen und einem Infrarotstrahler, in einer Querschnittsansicht im Bereich der Längenmitte des Metallrohres,
- **Figur 2**: einen Ausschnitt einer Ausführungsform eines Kühlgasführungsrohres zur wasserdichten Verbindung mit dem Strahlermodul gemäß Figur 1,
- **Figur 3**: eine verkleinerte, vollständige Darstellung des Kühlgasschlauches gemäß Figur 2, und
- **Figur 4**: eine Ausführungsform der erfindungsgemäßen Vorrichtung zur Entkeimung mit dem Strahlermodul von Figur 1 und mit dem Kühlgasführungsrohr von Figur 2 und 3.

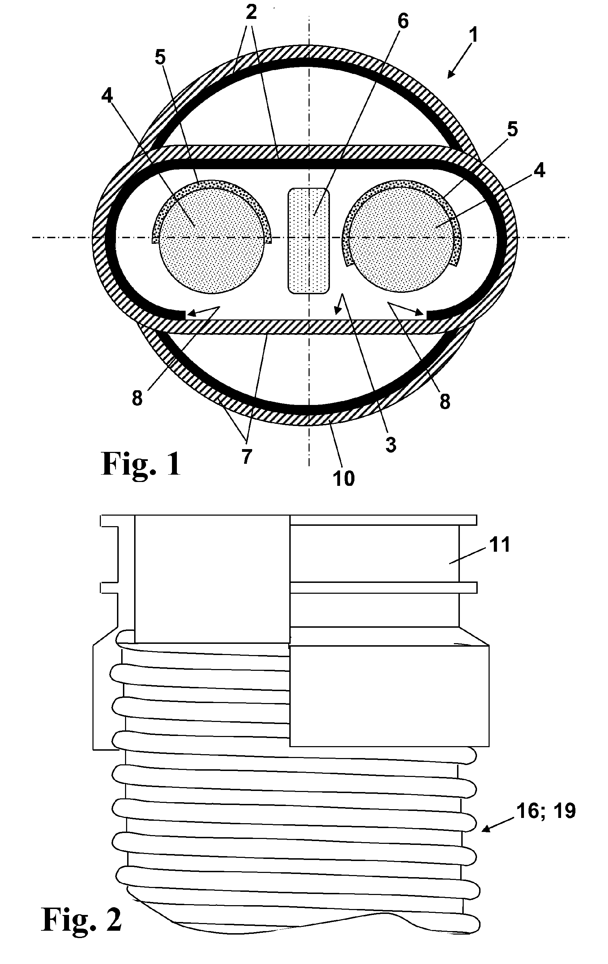

**Figur 1** zeigt schematisch ein Strahlermodul 1 zum Einsatz in der erfindungsgemäßen Vorrichtung zur Entkeimung von Packmaterial für Lebensmittel.

Das Strahlermodul 1 besteht im Ausführungsbeispiel aus einem etwa 1,5 mm dicken Metallrohr 2 aus Edelstahl, dessen beiderseitige Endbereiche 10 kreisrunden Querschnitt mit einem Außendurchmesser von 70 mm haben und dessen Mittelstück abgeflacht ist und einen länglich-ovalen Querschnitt hat, wie im Querschnitt von Figur 1 erkennbar. Das Metallrohr 2 einschließlich des abgeflachten Mittelstücks bildet einen Gehäuse-Innenraum, der begrenzt ist von zwei Flachseiten, die über gerundete Seitenwände miteinander verbunden sind. In eine der Flachseiten ist ein Strahlungsaustrittsfenster 3 eingeschnitten.

In dem Metallrohr-Gehäuse sind zwei UV-Strahler 4 angeordnet, die mit ihren Längsachsen parallel zueinander verlaufen. Die dem Strahlungsaustrittsfenster 3 abgewandte Seite der UV-Strahler 4 ist mit einer Reflektorschicht 5 aus opakem, diffus reflektierendem Quarzglas versehen. Mittig zwischen den beiden UV-Strahlern 4 ist ein Infrarotstrahler 6 angeordnet. Die Länge des Strahlungsaustrittsfensters 3 entspricht der Beleuchtungslänge der UV-Strahler 4.

Die UV-Strahler 4 sind Niederdruck-Amalgamstrahler mit einem Leuchtrohr aus Quarzglas, das einen Entladungsraum umschließt und das an beiden Enden mit Quetschungen verschlossen ist, durch die die Stromversorgung geführt ist. Innerhalb und an entgegengesetzten Enden des Leuchtrohres sind zwei wendelförmige Elektroden angeordnet. Der Entladungsraum ist mit einer Gasmischung aus Argon und Neon (50:50) gefüllt. Innerhalb des Entladungsraumes befindet sich außerdem ein Amalgamdepot. Der Niederdruck-Amalgamstrahler 4 wird mit einem im Wesentlichen konstanten Lampenstrom betrieben. Die Nominal-Leistung beträgt 200 W (bei einem nominalen Lampenstrom von 4,0 A), die Leuchtlänge 50 cm, der Strahler-Außendurchmesser 28 mm und er zeichnet sich durch eine Leistungsdichte von etwa 4 W/cm aus.

Der Infrarotstrahler 6 wird als externe Energiequelle zur Erwärmung der beiden UV-Strahler 4 verwendet. Er ist als sogenannter Zwillingsrohrstrahler ausgeführt, bei dem der Hüllkolben zwei parallel zueinander verlaufende Teilräume aufweist, die durch einen Mittelsteg voneinander getrennt sind. Innerhalb der Teilräume ist jeweils eine Heizwendel aus Wolfram in Argon-Schutzgas angeordnet. Die Nominal-Leistung des IR-Strahlers 6 (bei einem nominalen Lampenstrom von 8 A) beträgt 2.000 W. Die Außenmaße des Zwillingsrohrstrahlers 6 betragen 23 x 11 mm und die beleuchtete Lampenlänge entspricht etwa derjenigen der UV-Strahler 4. Er zeichnet sich durch eine Leistungsdichte von etwa 125 W/cm aus.

Das Modul aus Metallgehäuse 2, UV-Strahlern 4 und IR-Strahler 6 ist von einem 0,5 mm dicken UV-durchlässigen Polymerschlauch 7 (Fluorpolymer MFA) umgeben, der das Strahlungsaustrittsfenster 3 abdichtet. Der Polymerschlauch 7ist dabei über die gesamte Länge des Metallrohres 2 bis zu den Enden gezogen. Dabei bewirkt die Abflachung im Mittelstück des Metallrohres 2, das sich die Längskanten 8 im Bereich des eingeschliffenen Strahlungsaustrittsfensters 3 in einer gemeinsamen Ebene genau gegenüberliegen. Auf diese Weise wird für den Polymerschlauch 7 eine definierte Spannebene geschaffen, die Falten und Verwerfungen im Bereich des Strahlungsaustrittsfensters 3 verhindert.

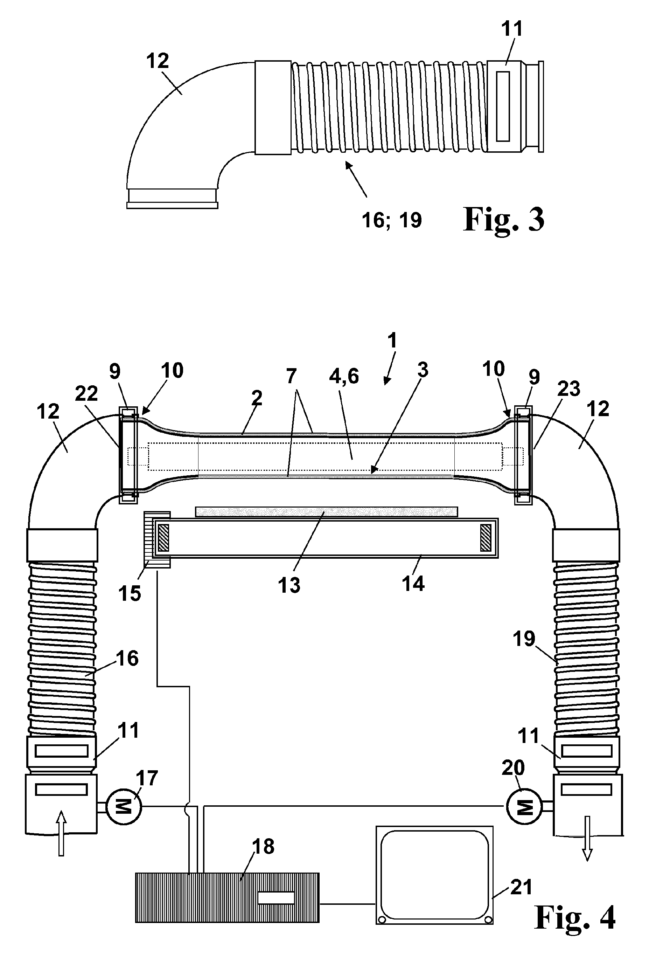

Die in der Figur 1 nicht erkennbaren stirnseitigen Enden des Metallgehäuses 2 sind offen und haben einen kreisrunden Querschnitt. Die offenen Gehäuse-Enden 10 dienen der Zuleitung und Ableitung eines Kühlgasstroms für eine Luftkühlung des Moduls 1. Zu diesem Zweck sind die Enden 10 mit Kühlgas-Spiralschläuchen 16; 19 aus Polyurethan verbunden, wie sie in den Figuren 2 und 3 dargestellt sind. An einem Ende ist an den Kühlgasschläuchen t jeweils eine zum Gehäuse 2 des Moduls 1 passende Muffe 12 angegossen (siehe Figur 3), die für eine wasserdichte Verbindung zum Gehäuse dient. Das andere Ende ist mit einem Verbindungsstück 11 für andere Anschlusselemente versehen, wie etwa Verlängerungsrohren oder Messgeräten. Die Muffen 12 zeigen eine gerade Erstreckung oder sie sind abgewinkelt wie in Figur 3 gezeigt.

Die Kühlgasschläuche dienen zur Zuführung (16) beziehungsweise Ableitung (19) eines Kühlgasstroms, insbesondere eines Luftstroms, zum und vom Strahlermodul 1 und gleichzeitig verlaufen darin die elektrischen Versorgungskabel für das Strahlermodul 1 und Datenleitungen. Somit benötigt das Strahlermodul 1 keine zusätzlichen Öffnungen für den Anschluss elektrischer Versorgungsleitungen.

Die Kühlgasspülung dient zur Kühlung/Temperierung der UV-Strahler 4 und deren Reflektoren 5. Im Ausführungsbeispiel wird Luft als Kühlgas eingesetzt. Wird stattdessen ein Inertgas verwendet, wie etwa Stickstoff, ermöglicht dies die Einhaltung einer nicht zündfähigen Atmosphäre im Gehäuseinnern, was zum Explosionsschutz beiträgt. Darüber hinaus verlängert die Kühlung auch die Lebensdauer des Polymerschlauches 7. So wurde bei einer UV-Bestrahlungszeit von mehr als 10.000 Stunden keine nennenswerte Veränderung der optischen Transmission und der mechanischen Stabilität des Polymerschlauches 7 festgestellt.

Eine weitere wesentliche Funktion des Kühlgasstroms im Sinne der Erfindung wird nachfolgend anhand der schematischen Darstellung der Gesamtvorrichtung in **Figur 4** zur Entkeimung näher erläutert.

Die Bestrahlungsvorrichtung 1 umfasst neben dem oben bereits erläuterten Strahlermodul 1 ein von einem Antriebsmotor 15 bewegtes Transportband 14, auf dem das zu bestrahlende Packmaterial 13 für Lebensmittel aufliegt und mittels diesem dem Strahlermodul 1 kontinuierlich zugeführt wird.

Das rohrförmige Metallgehäuse 2 des Strahlermoduls 1 ist seitlich mit den Kühlgasschläuchen 16; 19 verschlossen. Die beiden UV-Strahler 4 und der IR-Strahler 6 sind mit gestrichelter Umrandung angedeutet. Sie liegen in der Ansicht von Figur 4 in der Blattebene hintereinander und sind mit ihren Sockeln jeweils in Halteelemente eingesteckt, die am Ende 10 des Rohres montiert sind. Der Gaseinlass des Gehäuses 2 ist mit der Bezugsziffer 22 und der Gasauslass mit der Bezugsziffer 23 gekennzeichnet. Die angegossene Schlauchmuffe 12 umfängt dabei das runde Rohrende 10 des Metallgehäuses 2 mitsamt des darauf aufgezogenen Polymerschlauchs 7 und wird mittels einer Schlauchschelle 9 gegen den Außenumfang des Rohrendes gepresst.

Der Kühlgasschlauch 16 für die Zufuhr des Kühlluftstroms ist mit einem Massendurchflussregler 17 ausgestattet. Dieser ist mit einer Auswerte- und Regeleinheit 18 verbunden. Der Massenfluss des Kühlluftstroms liegt typischerweise im Bereich von 50 bis 100 m³/h.

Der Kühlgasschlauch 19 für die Abführung des Kühlluftstroms dient außerdem für die Zuführung aller elektrischer Kabel zum Betrieb des Strahlermoduls 1 sowie einer Signalleitung zu einem Temperatursensor, der innerhalb des StrahlermodulGehäuses 2 angeordnet ist und der die Oberflächentemperatur von einem der UV-Strahler erfasst. Der Kühlgasschlauch 19 ist mit einem MassendurchflussMesselement 20 für die abgeführte Kühlluft ausgestattet, der ebenfalls mit der Auswerte- und Regeleinheit 18 verbunden ist.

Die Auswerte- und Regeleinheit 18 ist mit dem besagten Temperatursensor verbunden, der die Oberflächentemperatur von einem der UV-Strahler 4 erfasst, als auch mit einem weiteren (in der Figur nicht dargestellten) Temperatursensor für die Messung der Oberflächentemperatur des zu bestrahlenden Packmaterials 13.

Die Auswerte- und Regeleinheit 18 dient zur Temperaturregelung, bei der die Oberflächentemperatur des UV-Strahlers 5 die Regelgröße ist. Stellgröße dafür ist der Kühlgasstrom, der von der Auswerte- und Regeleinheit 18 mittels des Massendurchflussreglers 17 eingestellt wird.

Gleichzeitig und kontinuierlich überwacht die Auswerte- und Regeleinheit 18 den Massendurchfluss des abgeführten Kühlluftstromes über das Messelement 20. Die Messdaten werden gleichzeitig auf einem Monitor 21 angezeigt. Wird eine Abweichung zwischen eingeleitetem und abgeführtem Luftstrom festgestellt, die höher ist als ein vorgegebener Grenzwert von 10% der eingeleiteten Luftströmung, so schaltet die Auswerte- und Regeleinheit 18 den Antrieb 15 für das Transportband 14 und das Strahlermodul 1 unverzüglich ab und gibt einen akustischen Alarm aus sowie eine entsprechende Anzeige auf dem Monitor 21. Bei besonders hohen Anforderungen an die Dichtheit des Strahlermoduls 1 ist alternativ oder ergänzend zur Messung des Massendurchflusses des Kühlluftstroms eine Gasdruckmessung vorgesehen, bei der der Gasdrucks des abgeführten Kühlluftstromes kontinuierlich überwacht und ausgewertet wird.

Durch die Verwendung der Kunststofffolie 7 zum dichten Abschluss des Strahlungseintrittsfensters 13 ist die erfindungsgemäße Entkeimungsvorrichtung wasserdicht. Obwohl auf ein kostspieliges Quarzglas-Hüllrohr für das Strahlermodul 1 verzichtet wird, ist die Vorrichtung trotz kostengünstiger Bauweise betriebssicher, da die Dichtheit der Abdichtung kontinuierlich kontrolliert wird.

## Patentansprüche

1. Vorrichtung zur Entkeimung mittels ultravioletter Strahlung, umfassend einen UV-Strahler (4), der in einem Gehäuse (2) aus Metall oder aus Kunststoff angeordnet ist, das ein Strahlungsaustrittsfenster (3) für die Emission von UV-Strahlung aufweist, welches von einer für ultraviolette Strahlung durchlässigen Polymerfolie (7) bedeckt ist, **dadurch gekennzeichnet, dass** das Gehäuse (2) einen Gaseinlass (22) für die Einleitung eines Kühlgasstromes sowie einen Gasauslass (23) für die Ableitung des Kühlgasstromes aufweist, und dass der Gasauslass (23) mit einer Messeinrichtung (17; 18, 20, 21) zur Messung von Druck, Massenstrom und/oder Volumenstrom des abgeleiteten Kühlgasstromes verbunden ist.

2. Vorrichtung nach Anspruch 1, **dadurch gekennzeichnet, dass** die Messeinrichtung (17; 18, 20, 21) einen mit dem Gaseinlass (22) verbundenen, ersten Sensor (17) und einen mit dem Gasauslass (23) verbundenen, zweiten Sensor (20) umfasst.

3. Vorrichtung nach Anspruch 2, **dadurch gekennzeichnet, dass** mindestens der erste Sensor (17) als Massendurchflussregler ausgeführt ist.

4. Vorrichtung nach Anspruch 1 oder 2, **dadurch gekennzeichnet, dass** die Messeinrichtung (17; 18, 20, 21) eine Anzeige- oder Alarmeinrichtung (21) umfasst.

5. Vorrichtung nach einem der vorhergehenden Ansprüche, **dadurch gekennzeichnet, dass** die Messeinrichtung (17; 18, 20, 21) eine Auswerte- und Abschalteinrichtung (18) umfasst.

6. Vorrichtung nach einem der vorhergehenden Ansprüche, **dadurch gekennzeichnet, dass** der UV-Strahler Teil einer innerhalb des Gehäuses (2) angeordneten Lampeneinheit ist, die zwei UV-Strahler (4) und einen IR-Strahler (6) aufweist.

7. Vorrichtung nach Anspruch 6, **dadurch gekennzeichnet, dass** mindestens die UV-Strahler (4) auf ihrer dem Strahlungsaustrittsfenster (3) abgewandten Seite mit einem Reflektor (5) versehen sind.

8. Vorrichtung nach Anspruch 6 oder 7, **dadurch gekennzeichnet, dass** das Gehäuse (2) im Bereich des Strahlungsaustrittsfenster (3) einen länglichovalen oder rechteckigen Querschnitt hat.

9. Vorrichtung nach einem der vorhergehenden Ansprüche, **dadurch gekennzeichnet, dass** die Messeinrichtung (17; 18, 20, 21) einen Temperatursensor zur Erfassung der Temperatur des UV-Strahlers (4) umfasst.

10. Vorrichtung nach einem der vorhergehenden Ansprüche, **dadurch gekennzeichnet, dass** der Gaseinlass (22) mit einer Temperiervorrichtung verbunden ist, mittels welcher der Kühlgasstrom temperierbar ist.

11. Vorrichtung nach einem der vorhergehenden Ansprüche, **dadurch gekennzeichnet, dass** an den Gaseinlass (22) und/oder an den Gasauslass (23) ein Kühlgasführungsrohr (19) angeschlossen ist, durch das Versorgungsoder Datenleitungen hindurchgeführt sind.
